(19) Europäisches Patentamt
European Patent Office
Office européen des brevets

(11) **EP 3 100 720 A1**

(12) **EUROPEAN PATENT APPLICATION**

(43) Date of publication:
**07.12.2016 Bulletin 2016/49**

(51) Int Cl.:
***A61K 9/16*** *(2006.01)*      ***A61K 9/20*** *(2006.01)*
***A61K 31/00*** *(2006.01)*

(21) Application number: **16171928.1**

(22) Date of filing: **30.05.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **05.06.2015 TW 104118277**

(71) Applicant: ANXO Pharmaceutical Co., Ltd.
Taipei (TW)

(72) Inventors:
• HUANG, Chen-Ming
Taipei City (TW)
• CHUANG, Li-Chuan
Hsinchu City (TW)
• LI, Chih-Hung
Miaoli County (TW)

(74) Representative: **Becker Kurig Straus**
**Patentanwälte**
**Bavariastrasse 7**
**80336 München (DE)**

(54) **SUSTAINED RELEASE PHARMACEUTICAL COMPOSITION AND PREPARATION METHOD THEREOF**

(57) Provided is a method for preparation of a sustained released pharmaceutical composition, wherein an active ingredient and a cellulose derivative dissolved in a lower alkyl alcohol solvent are processed with granulation such that the PEO does not need to be processed with granulation or sizing to achieve uniform drug distribution. Besides, the specific range of the viscosity of the cellulose derivate provides better uniformity to the sustained release pharmaceutical composition derived from the above preparation method.

FIG. 1

Printed by Jouve, 75001 PARIS (FR)

**Description**

CROSS REFERENCE

This application claims priority to Taiwan Patent Application Serial No.104118277, filed on June 5, 2015, the content of which is hereby incorporated by reference in its entirety.

1. Field of the Invention

The present invention relates to a pharmaceutical composition, particularly to a sustained release pharmaceutical composition; the present invention also relates to a method for preparation of the above pharmaceutical composition, particular to a preparation method to obtain the sustained release pharmaceutical composition without granulating or sizing the polyalkylene oxide.

2. Description of the Prior Arts

Drug release of sustained release oral dosage form is often affected by the food in the gastrointestinal tract, such that the dosage form is difficult to maintain and the release rate is unstable. The retention capacity of the dosage form in the gastrointestinal tract is poor, such that the drug is mostly released in the upper gastrointestinal tract, including the stomach and the small intestine, and shortening the drug absorption time.
In view of this, Taiwan patent number 393320 discloses an oral hydrogel controlled release pharmaceutical tablet, wherein polyethylene oxide (PEO) is used as high molecular of hydrogel-forming base and then together with suitable hydrophilic base, such as polyethylene glycol (PEG). The tablet would completely gelatinize during its staying in the stomach and the small intestine of the upper gastrointestinal tract. The colloidal PEO has great plasticity after gelation, and the surface erosion rate of the dosage form is moderate and less susceptible to the impact of the food, thereby stably releasing the drug. When the dosage form is moved downward to the lower gastrointestinal tract, the active ingredient of the dosage form still can be released in the colon. However, in the preparation of the above mentioned patent, the active ingredient, the hydrophilic base and hydrogel-forming base are usually directly mixed and then proceed tableting (direct tableting), or the ingredients are directly mixed and then tableted after wet/dry granulation. In some embodiments, the active ingredient and portions of hydrophilic base are further dissolved in alcohol solvent or its mixture with water, and the solution is sprayed and dried to obtain a spray-dried product. The spray-dried product is further mixed with the remaining hydrophilic base and hydrogel-forming base for tableting. The steps of direct tableting and wet/dry granulation described above is not suitable for tableting due to the poor fluidity of the powder after mixing and granulating, and the uniformity of drug content would be obviously diminished while preparing the pharmaceutical composition containing a low dose of active ingredient. Besides, the manufacture steps of producing the spray-dried product then mixing and tableting are too complicated and time consuming.
Taiwan issued patent number I307632 further improves the oral hydrogel controlled release pharmaceutical tablet and the process thereof provided by Taiwan issued patent number 393320. The invention of patent I307632 also uses the PEO as hydrogel-forming base. In order to solve the problem of unequal distribution of particle sizes, non-uniformity of drug content and poor powder fluidity after mixing and granulation in the patent number 393320, the active ingredient and the hydrophilic base are dispersed and dissolved in the water to form a suspension, and then the PEO powder undergoes a sizing process by spraying the suspension on it. During the process of sizing, the hydrophilic base is used as sizing agent. Although the spraying and sizing process in the patent number I307632 improves the disadvantages of non-uniformity of drug content and poor PEO powder fluidity after granulation, the condition of granulation and sizing are difficult to control due to the physicochemical properties of PEO. It requires tedious attempts to find the suitable condition of granulation and sizing. It also means that the granulation and sizing of PEO needs more sophisticated regulation during the stage of mass production. The disadvantages of Taiwan patent number I307632 are that the spraying and sizing process needs to control many factors including the amount of the suspension containing the active ingredient, the spraying time and the spraying temperature, the drying time and the drying temperature, and the frequency of the vibration, etc. The manufacture is complicated and the equipment for spraying and sizing (such as fluidized bed granulator) is expensive and needs large space, the equipment is hard to clean after the process due to high viscosity of PEO, thereby being unfavorable for large scale preparation.
European patent number EP1205190 teaches that PEO as the component of the sustained release base drug would accelerate dissolution after exposure to light, leading to increased release rate of the active ingredient and changes of release profile. Furthermore, the side chain of the PEO contains a lot of ether bond such that PEO is easily degraded due to the oxygen attack, and heavy metal ions, oxidants and ultraviolet will accelerate the oxidative degradation process. As a result, the pharmaceutical composition using PEO as sustained release base can achieve sustained release effect, but PEO is expensive and the physicochemical properties of PEO is unstable and susceptible to oxidation, thereby increasing risk factors to the stability and storage of the drug.

In view of the disadvantages of poor fluidity, unequal distribution of particle sizes, non-uniformity of drug content, complicated manufacture process, high time consumption, and cleaning difficulty of the equipment in the prior arts, the objective of the present invention is to provide a method for preparation of a sustained release pharmaceutical composition, comprising granulating the active ingredient and the cellulose derivative with a lower alkyl alcohol solvent, and then the PEO does not need to process granulation or sizing to achieve uniformity of drug content.

To achieve the above purpose of the present invention, the objective of the invention is to provide a method for preparation of a sustained release pharmaceutical composition, comprising steps of: mixing and granulating an active ingredient, a lower alkyl alcohol solvent having a concentration not lower than 80% (w/v) and a cellulose derivate to obtain multiple granulated powders; drying the multiple granulated powders to remove the lower alkyl alcohol solvent; mixing the dried multiple granulated powders, polyalkylene oxide and a lubricant to form a mixture; compressing the mixture to obtain a sustained release pharmaceutical composition.

Preferably, the active ingredient comprises an agent substance proposed to be formulated in a sustained release dosage form.

Preferably, the active ingredient comprises an anti-benign prostatic hyperplasia (BPH) agent, an anti-urinary tract disease agent, an antihypertensive agent, an antipsychotic agent, an anti-inflammatory agent, an antipyretic agent, an analgesic agent, an anti-metabolic disorder agent, an anti-cardiovascular disease agent, an immunosuppressive agent, an anti-tumor agent, or any combination thereof.

Preferably, the active ingredient comprises tamsulosin, mirabegron, quetiapine, or a pharmaceutically acceptable salt thereof.

Preferably, in the step of mixing and granulating the active ingredient, the lower alkyl alcohol solvent having the concentration not lower than 80% (w/v) and the cellulose derivate, the active ingredient is dissolved in the lower alkyl alcohol solvent having the concentration not lower than 80% (w/v) followed by mixing and granulation with the cellulose derivate.

Preferably, in the step of mixing and granulating the active ingredient, the lower alkyl alcohol solvent having the concentration not lower than 80% (w/v) and the cellulose derivate, the active ingredient is mixed with the cellulose derivate followed by granulating with the lower alkyl alcohol solvent having the concentration not lower than 80% (w/v).

Preferably, the method after the step of compressing the mixture further includes film coating the compressed tablet.

The "lower alkyl alcohol solvent having the concentration not lower than 80% (w/v)" refers to that the concentration of the lower alkyl alcohol solvent is from 80% (w/v) to 100% (w/v); preferably, the concentration of the lower alkyl alcohol solvent is from 90% (w/v) to 99.5% (w/v).

Besides, the lower alkyl alcohol solvent includes, but is not limited to, methanol, ethanol, propanol, isopropanol, butanol, or any combination thereof.

In the method for preparation of the present invention, the amount of the lower alkyl alcohol solvent is adjusted depending on the amount of powder for granulation during the step of mixing and granulating the active ingredient, the lower alkyl alcohol solvent having the concentration not lower than 80% (w/v) and the cellulose derivate; preferably, the weight ratio of the lower alkyl alcohol solvent and the powder for granulation is from 1:100 to 100:1. More preferably, the weight ratio of the lower alkyl alcohol solvent and the powder for granulation is from 1:10 to 10:1. Most preferably, the weight ratio of the lower alkyl alcohol solvent and the powder for granulation is from 1:5 to 5:1.

Preferably, the cellulose derivate includes, but is not limited to, hydroxypropylcellulose (HPC), hypromellose (HPMC), methyl cellulose (MC), hydroxyethyl cellulose (HEC), ethyl cellulose (EC), or any combination thereof.

Preferably, the cellulose derivate is hypromellose with a viscosity ranging from 50 millipascal seconds (mPa·s) to 100,000 mPa·s at 1% w/v, 20°C.

Preferably, the polyalkylene oxide includes, but is not limited to, polyethylene oxide (PEO), polypropylene oxide (PPO), poly(propylene oxide-ethylene oxide), or any combination thereof.

Preferably, the polyalkylene oxide is polyethylene oxide with a molecular weight ranging from 500,000dalton (Da) to 7,000,000 Da.

Preferably, the method after the step of mixing and granulating the active ingredient, the lower alkyl alcohol solvent having the concentration not lower than 80% (w/v) and the cellulose derivate further comprises adding a solubilizer.

More preferably, the solubilizer includes, but is not limited to, polyethylene glycol (PEG), docusate sodium, sodium lauryl sulfate, medium chain triglyceride, polyvinylpyrrolidone (PVP), xylitol, D-fructose, urea, acetylamine, polyoxyl 40 hydrogenated castor oil, polyoxyl 60 hydrogenated castor oil, poloxamer 407, benzoic acid, sodium benzoate, salicylic acid, sodium salicylate, or any combination thereof.

In another aspect, the present invention relates to a sustained release pharmaceutical composition obtained from the above preparation method.

In another aspect, the present invention relates to a sustained release pharmaceutical composition, which is made by uniformly mixing and tableting the ingredients consisting of an active ingredient; a cellulose derivate having viscosity from 50 mPa·s to 100,000 mPa·s and being hard to dissolve in a lower alkyl alcohol solvent having the concentration not lower than 80% (w/v); the lower alkyl alcohol solvent having the concentration not lower than 80% (w/v) in an amount of less than 5% in weight (wt%) based on the total weight of the sustained release pharmaceutical composition, wherein the lower alkyl alcohol solvent is used for mixing and granulating with the active ingredient and the cellulose derivate to obtain powder particles without agglomerating; a polyalkylene oxide having an average molecular weight above 500,000 Da; and a lubricant.

Preferably, the polyalkylene oxide is un-granulated or un-sized.

Preferably, the ratio of the cellulose derivate and the polyalkylene oxide of the sustained release pharmaceutical composition is from 1:100 to 100:1; more preferably, the ratio is from 1:10 to 10:1; most preferably, the ratio is from 1:5 to 5:1.

Preferably, based on the total weight percent (wt%) of the sustained release pharmaceutical composition as mentioned above, the weight percent of the active ingredient is from 0.01 wt% to 25 wt%; the weight percent of the cellulose derivate having the viscosity from 50 mPa·s to 100,000 mPa·s is from 1 wt% to 50 wt%; the weight percent of the lower alkyl alcohol solvent having the concentration not lower than 80% (w/v) is from 0 wt% to 5 wt%; the weight percent of the polyalkylene oxide having an average molecular weight above 500,000 Da is from 30 wt% to 95 wt%; and the weight percent of the lubricant is from 0.1 wt% to 5 wt%; wherein the cellulose derivate is hard to dissolve in the lower alkyl alcohol solvent having the concentration not lower than 80% (w/v); wherein the lower alkyl alcohol solvent is used for mixing and granulating with the active ingredient and the cellulose derivate to obtain the powder particles without agglomerating.

More preferably, the weight percent of the lower alkyl alcohol solvent having the concentration not lower than 80% (w/v) in the sustained release pharmaceutical composition is from 0 wt% to 2 wt%; most preferably, the weight percent is from 0 wt% to 1 wt%.

More preferably, the sustained release pharmaceutical composition further comprises a solubilizer, which the weight percent is from 0 wt% to 10 wt%.

As HPMC is hard to dissolve in the high concentration alcohol solvent, the method of spraying granulation will be chosen when having the active ingredient dissolved in the alcohol solvent followed by granulating with HPMC in the prior arts, rather than mixing and granulating the drug containing alcohol solvent and the HPMC powder directly as the present invention. The preparation method of the present invention can avoid time, space, and cost required for spraying granulation. Besides, the specific range of the viscosity of the cellulose derivate of the present invention provides better uniformity of drug content to the sustained release pharmaceutical composition.

Other objectives, advantages and novel features of the invention will become more apparent from the following detailed description when taken in conjunction with the accompanying drawings.

IN THE DRAWINGS:

Fig. 1 shows a line chart of dissolution rates of the tablets of the preparations A, B, E and F made by a preparation method of the present invention at 0 hour, 3 hours, 6 hours, 8 hours and 10 hours.

Fig. 2 shows a line chart of dissolution rates of the tablets of the preparations C and D made by the preparation method of the present invention at 0 hour, 3 hours, 6 hours, 8 hours and 10 hours.

Fig. 3 shows a line chart of dissolution rates of the tablets of the preparations A and C made by the preparation method of the present invention at 0 hour, 3 hours, 6 hours, 8 hours and 10 hours.

Fig. 4 shows a line chart of dissolution rates of the tablet of the preparation A at 0 hour to 24 hours after storage for 0 month or 3 months.

Fig. 5 shows a line chart of dissolution rates of the tablet of the preparation E at 0 hour to 24 hours after storage for 0 month or 3 months.

**Preparation of 1,000 units of sustained release dosage form**

Table 1. Formulation [unit: gram (g)]

| | Hypromellose 50 | | | Hypromellose 4000 | Hypromellose 100000 | |
| --- | --- | --- | --- | --- | --- | --- |
| Preparation | A | B | C | D | E | F |
| Tamsulosin HCl | 0.4 | 0.4 | 0.4 | 0.4 | 0.4 | 0.4 |

(continued)

|  | Hypromellose 50 | | | Hypromellose 4000 | Hypromellose | 100000 |
|---|---|---|---|---|---|---|
| Preparation | A | B | C | D | E | F |
| PEG 6000 | 4 | 4 | 4 | 4 | 4 | 4 |
| Hypromellose | 45 | 45 | 140.5 | 140.5 | 45 | 45 |
| POLYOX™ WSR 303 | 191 | 141 | 95.5 | 95.5 | 191 | 141 |
| Magnesium stearate | 4.6 | 4.6 | 4.6 | 4.6 | 4.6 | 4.6 |
| Total weight | 245 | 195 | 245 | 245 | 245 | 195 |

As shown in Table 1, tamsulosin hydrochloride (HCl) and PEG 6000 were dissolved in the proper amount of 95% ethanol to prepare an active ingredient-containing ethanol solution. The active ingredient-containing ethanol solution was mixed with HPMC with different viscosities, respectively Hypromellose 50 (manufacturer: Shin-Etsu Chemical Co., Ltd., category: Metolose® 60SH Grades 60SH-50) with the viscosity of 50 millipascal seconds (mPa·s), Hypromellose 4000 (manufacturer: The Dow Chemical Company, category: METHOCEL™ K4m Premium Hydroxypropyl Methylcellulose ID34693) with the viscosity of 4,000 mPa·s and Hypromellose 100000 (manufacturer: Shin-Etsu Chemical Co., Ltd., category: Metolose® 90SH Grades 90SH-100000SR) with the viscosity of 100,000 mPa·s, and then was granulated by the granulator to obtain granular powder. The granular powder was dried by dryer at 65°C to remove the ethanol, and then the dried granular powder was sieved through a proper sieve. Water-soluble polyethylene oxide (PEO) polymers such as POLYOX™ WSR 303(manufacturer: The Dow Chemical Company, category: POLYOX™ WSR 303-NF Grade), magnesium stearate and the dried granular powder after sieving were uniformly mixed to obtain a final mixture. The final mixture was compressed by tablet press into 195 milligrams (mg) or 245 mg of uncoated tablet (semi-finished). In the preferred embodiment, the uncoated tablet was further film coated.

**Example 1 Release rate test of the sustained release dosage form**

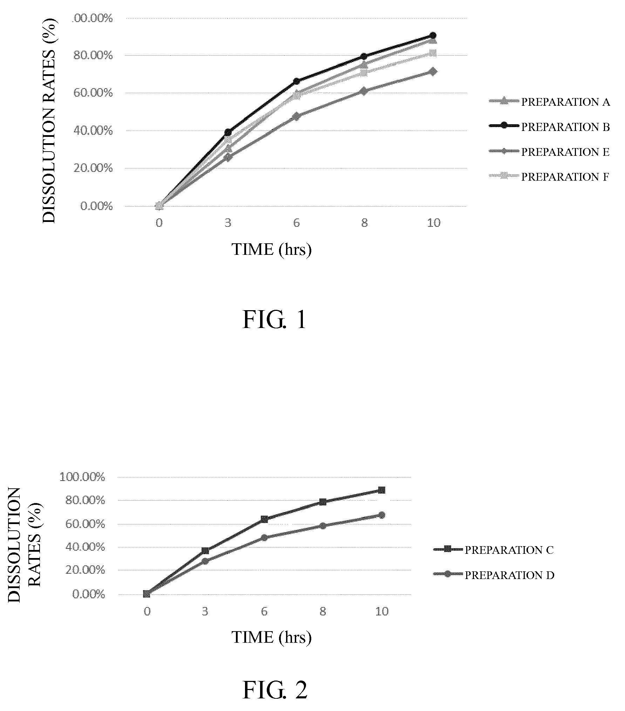

The tablets of preparations A, B, C, D, E and F were undergone a dissolution test in a dissolution apparatus [United States Pharmacopeia (USP) Apparatus II] at 100 rpm, using in 500 milliliters (mL) of 0.1N hydrochloride. Each tablet was tested for the dissolution rate at 0 hour, 3 hours, 6 hours, 8 hours and 10 hours, and the test result was as shown in table 2, Fig. 1, Fig. 2 and Fig. 3.

Table 2. Release rate of sustained release dosage form

| Time (hr) | Preparation A | Preparation B | Preparation C | Preparation D | Preparation E | Preparation F |
|---|---|---|---|---|---|---|
| 0 | 0.00% | 0.00% | 0.00% | 0.00% | 0.00% | 0.00% |
| 3 | 30.97% | 39.27% | 36.70% | 27.86% | 26.13% | 35.44% |
| 6 | 59.72% | 66.08% | 63.77% | 48.27% | 47.24% | 58.27% |
| 8 | 75.35% | 79.53% | 78.68% | 58.27% | 60.81% | 70.93% |
| 10 | 88.58% | 90.51% | 88.54% | 67.83% | 71.42% | 81.26% |

As shown in Fig. 1, the ratio of each ingredient of the preparations A and E of the present invention was the same, but the viscosity of preparation A using Hypromellose 50 was lower than the viscosity of preparation E using Hypromellose 100000 such that the release rate of preparation A was faster than preparation E at the same time point. Similarly, the comparison of the release rate between the preparations B and F, preparations C and D (as shown in Fig. 2) also had the same result. As above mentioned, the release rate was slower while the viscosity of the cellulose derivation used was higher. Therefore, the release rate can be controlled by selecting a high or low viscosity of the cellulose derivation.

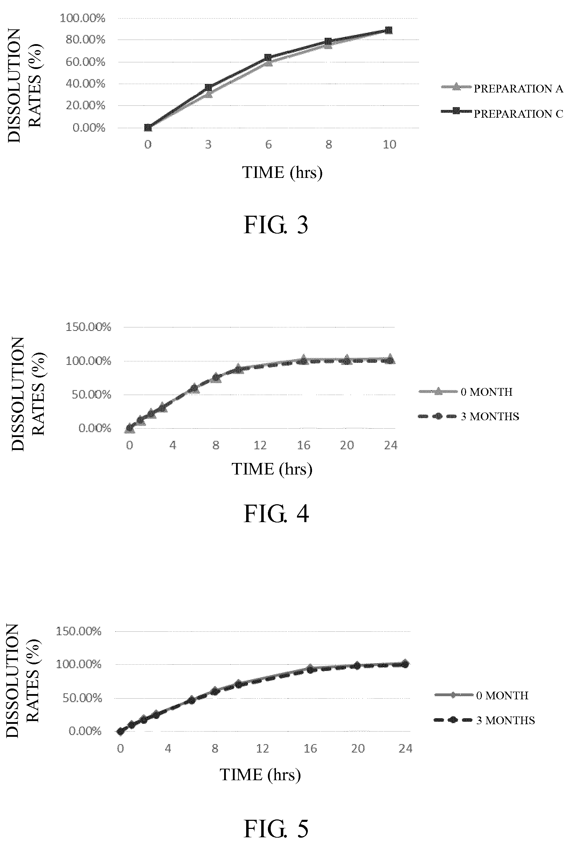

Furthermore, as shown in Fig. 1, the release rate of the preparation A of the present invention was lower than the preparation B because the content of POLYOX™ WSR 303 of the preparation A was higher than the preparation B. It also means that under the same ingredients of each preparation, the content of the polyalkylene oxide was higher, and the release rate was slower. However, upon further observation of the preparation C in Fig. 3, if the total weight of the tablet was 245 mg, same as preparation A, with different ratios of the cellulose derivation and polyalkylene oxide (such as increasing the content of Hypromellose 50 and lowering the content of POLYOX™ WSR 303), the release rate of preparation C can be nearly the same as preparation A. As a result, the composition of the present invention can increase the ratio of the cellulose derivation for decreasing the use of the polyalkylene oxide, thereby decreasing the uncertain factors and the preparation method inconvenience derived from the polyalkylene oxide.

**Example 2 Powder particle size distribution and fluidity analysis**

1. Particle size distribution: the final mixtures of preparations A and E obtained from the preparation method were respectively and randomly sampled and quantitated, and the final mixtures were placed into a digital sieve shaker (manufacturer: Endecotts LTD., category: Octagon Digital) and shaken for 20 minutes with a frequency of 3600 vibrations per minute and the amplitude of mode 5. The preparations A and E were observed for retention status in various sieve meshes with different pore size.

2. Angle of repose: the final mixtures of preparations A and E after quantitation were respectively and naturally fallen on the measurement platform by continuous vibration through a powder characteristics tester (manufacturer: Tsutsui Scientific, category: ABD-100), and the biggest angle was measured between the horizontal surface and the free surface of the powder on the platform forming a cone-shaped pile under static balance. The preferred angle of repose was below 40°, the smaller the angle of repose, the smaller the friction and the better the fluidity.

3. Bulk density: the final mixtures of preparations A and E after quantitation were respectively and left naturally falling into a container with an internal volume of 100 mL by continuous vibration through a powder characteristics tester, and the density was measured after the powder naturally filled the container.

4. Tap density: the final mixtures of preparations A and E after quantitation were respectively placed into a container with an internal volume of 100 mL and the powder-loaded container was continuously vibrated through a powder characteristics tester to condense the powder, and the density was measured under the compact powder.

5. Compressibility: based on the above bulk density and tap density, the compressibility was calculated by the following formula (I). The preferred compressibility was equal to or smaller than 20%, the smaller the compressibility, the better the powder fluidity.

$$\text{Compressibility (\%)} = (\text{Tap density} - \text{Bulk density})/\text{Tap density} \times 100 \quad \text{Formula (I)}$$

Table 3. The fluidity test result

| The fluidity test of the final mixtures | | Preparation A | Preparation E |
|---|---|---|---|
| Particle size distribution (%) | 75 μm to 425 μm | 79% | 80.3% |
| | < 75 μm | 20.4% | 19% |
| Angle of repose (°) | | 37.0 | 36.2 |
| Bulk density (mL/g) | | 0.356 | 0.356 |
| Tap density (mL/g) | | 0.444 | 0.434 |
| Compressibility (%) | | 19.81 | 17.19 |

As shown in table 3, 80% of the particle size of the final mixtures of the preparations A and E of the present invention distributed in the range from 75 micrometers (μm) to 425 μm, and the particle size smaller than 75μm was less than 20%. Therefore, the preparations A and E had little adhesion and scattering during the tableting process.

Besides, as shown in table 3, the angles of repose of the final mixtures of preparations A and E of the present invention were equal to or less than 37° and the compressibility was smaller than 20% such that the final mixtures of preparations A and E of the present invention have great powder fluidity. Furthermore, also as shown in table 3, although the viscosities of the cellulose derivatives used by the preparations A and E were different, the powder fluidities of the final mixtures do not decrease. The reason was that the preparation method of the present invention uses high concentration of alcohol solvent and cellulose derivate to granulation, and the granulation process does not produce the same sticky particle agglomerates as granulated by water but form fluffy powder because the cellulose derivate was hard to dissolve in the high concentration alcohol. Even high viscosity of cellulose derivate is used, the powder still can maintain great fluidity.

**Example 3 Uniformity test of drug content**

The final mixtures of preparations A and E obtained from the preparation method were respectively and randomly sampled for 245 mg process the active ingredient content analysis to determine the uniformity of the active ingredient content between intermediate (the final mixtures) and semi-finished product (uncoated tablets) during preparation. The sample analysis result as shown in table 4 was based on the active ingredient content of a single unit of a tablet.

Table 4. Uniformity result of each sample and its uncoated tablet

| Content of active ingredient (unit: %) | | |
|---|---|---|
| | Preparation A | Preparation E |
| Final mixture | 101.72%<br>RSD: 0.61% | 102.12%<br>RSD: 0.86% |
| Uncoated tablet | 101.20%<br>RSD: 0.77% | 103.23%<br>RSD: 0.80% |

As shown in table 4, the drug contents of the final mixtures (intermediate) and uncoated tablets (semi-finished product) of preparations A and E obtained from the preparation method fell within the narrow range of 90% to 110% and had the relative standard deviation (RSD) less than 3%, meaning that the content uniformity of each preparation of the present invention was significantly better than the USP criteria, having the limits of 85% to 115% and the RSD less than 6%. Based on the above data, we can infer that in the preparation method of the present invention, even the polyalkylene oxide with high viscosity and poor fluidity does not be processed with sizing or granulating, the drug can also be uniformly distributed, thereby solving the disadvantages of poor powder fluidity, being inappropriate for tableting, or poor uniform distribution of drug in the prior arts.

**Example 4 Stability test**

1. Stability test of the active ingredient

The tablets obtained from the preparations A and E were placed in a chamber with constant temperature of $40\pm2°C$ and humidity of $75\pm5\%$ for 0, 1, 2 and 3 months, and the active ingredient content in the tablet was measured to determine the degradation thereof in the dosage form of the present invention under the above condition, and the result was as shown in table 5.

Table 5. Stability test result

| Content of active ingredient (unit: %) | | |
|---|---|---|
| Time (month) | Preparation A | Preparation E |
| 0 | 101.20%<br>RSD: 0.77% | 103.23%<br>RSD: 0.80%% |
| 1 | 102.36%<br>RSD: 0.60% | 103.87%<br>RSD: 1.01% |
| 2 | 101.36%<br>RSD:0.38% | 102.40%<br>RSD: 0.86% |
| 3 | 102.05%<br>RSD:0.43% | 102.40%<br>RSD: 0.91% |

As shown in table 5, after being stored under high temperature and high humidity condition for 1 month to 3 months, the active ingredient in the tablets obtained the preparations A and E did not significantly degrade and the active ingredient content was still inside the standard range of 95% to 105%, demonstrating that the dosage form of the present invention has high stability.

2. Dissolution stability test

The tablets obtained from the preparations A and E were placed in a chamber with constant temperature of 40±2°C and humidity of 75±5% for 0 or 3 months, and then were undergone a dissolution test in a dissolution apparatus [United States Pharmacopeia (USP) Apparatus II] at 100 rpm, using 500 mL of 0.1N HCl. Each tablet was tested for the dissolution rates at 0, 1, 2, 3, 6, 8, 10, 16, 20 and 24 hours, and the results were as shown in table 6, Fig. 4 and Fig. 5.

### Table 6. Dissolution test result

| Dissolution rate of the active ingredient (unit: %) | | | | |
|---|---|---|---|---|
| Time | Preparation A | | Preparation E | |
| Month(s) / Hour(s) | 0 | 3 | 0 | 3 |
| 0 | 0.00% | 0.00% | 0.00% | 0.00% |
| 1 | 12.03% | 12.02% | 10.70% | 9.43% |
| 2 | 21.54% | 21.17% | 18.56% | 16.83% |
| 3 | 30.97% | 29.77% | 26.13% | 24.46% |
| 6 | 59.72% | 59.76% | 47.24% | 46.24% |
| 8 | 75.35% | 75.51% | 60.81% | 58.54% |
| 10 | 88.58% | 87.06% | 71.42% | 68.80% |
| 16 | 101.72% | 98.79% | 94.61% | 90.80% |
| 20 | 102.64% | 99.19% | 98.69% | 97.52% |
| 24 | 103.48% | 99.93% | 102.24% | 99.16% |

According to the dissolution rate of each time point shown in table 6, we can know that the tablet of preparations A and E of the present invention can continuously release the drug contents for 16 hours to 24 hours. As shown in Fig.4 and Fig. 5, even the tablets of preparations A and E were treated with high temperature and high humidity for 3 months, the dissolution rates thereof were found no significant difference with the untreated group (0 hour). The treated tablets still can exhibit the desired release profile. Therefore, the dosage form of the present invention has high stability.

Even though numerous characteristics and advantages of the present invention have been set forth in the foregoing description, together with details of the structure and features of the invention, the disclosure is illustrative only. Changes may be made in the details, especially in matters of shape, size, and arrangement of parts within the principles of the invention to the full extent indicated by the broad general meaning of the terms in which the appended claims are expressed.

**Claims**

1. A method for preparation of a sustained release pharmaceutical composition, **characterized in that** the method for preparation of a sustained release pharmaceutical composition comprises steps of:

    mixing and granulating an active ingredient, a lower alkyl alcohol solvent having a concentration not lower than 80% (w/v) and a cellulose derivate to obtain multiple granulated powders;
    drying the multiple granulated powders to remove the lower alkyl alcohol solvent;
    mixing the dried multiple granulated powders, polyalkylene oxide and a lubricant to form a mixture which is uniform;

compressing and tableting the mixture to obtain a sustained release pharmaceutical composition.

2. The method as claimed in claim 1, wherein the active ingredient comprises an agent proposed to be formulated in a sustained release dosage form.

3. The method as claimed in claim 1, wherein the active ingredient comprises an anti-benign prostatic hyperplasia (BPH) agent, an anti-urinary tract disease agent, an antihypertensive agent, an antipsychotic agent, an anti-inflammatory agent, an antipyretic agent, an analgesic agent, an anti-metabolic disorder agent, an anti-cardiovascular disease agent, an immunosuppressive agent, an anti-tumor agent, or any combination thereof.

4. The method as claimed in claim 1, wherein the active ingredient comprises tamsulosin, mirabegron, quetiapine, or a pharmaceutically acceptable salt thereof.

5. The method as claimed in claim 1, wherein in the step of mixing and granulating the active ingredient, the lower alkyl alcohol solvent having the concentration not lower than 80% (w/v) and the cellulose derivate, the active ingredient is dissolved in the lower alkyl alcohol solvent having the concentration not lower than 80% (w/v) followed by mixing and granulation with the cellulose derivate.

6. The method as claimed in claim 1, wherein in the step of mixing and granulating the active ingredient, the lower alkyl alcohol solvent having the concentration not lower than 80% (w/v) and the cellulose derivate, the active ingredient is mixed with the cellulose derivate followed by granulating with the lower alkyl alcohol solvent having the concentration not lower than 80% (w/v).

7. The method as claimed in claim 1, wherein the method after the step of compressing the mixture further includes film coating the compressed tablet.

8. The method as claimed in claim 1, wherein the lower alkyl alcohol solvent having the concentration not lower than 80% (w/v) comprises methanol, ethanol, propanol, isopropanol, butanol, or any combination thereof.

9. The method as claimed in claim 1, wherein the cellulose derivate comprises hydroxypropylcellulose (HPC), hypromellose (HPMC), methyl cellulose (MC), hydroxyethyl cellulose (HEC), ethyl cellulose (EC), or any combination thereof.

10. The method as claimed in claim 1, wherein the cellulose derivate is hypromellose with the viscosity ranging from 50 millipascal seconds (mPa·s) to 100,000 mPa·s at 1% w/v, 20°C.

11. The method as claimed in claim 1, wherein the polyalkylene oxide comprises polyethylene oxide (PEO), polypropylene oxide (PPO), poly(propylene oxide-ethylene oxide), or any combination thereof.

12. The method as claimed in claim 1, wherein the polyalkylene oxide is polyethylene oxide with the molecular weight ranging from 500,000dalton (Da) to 7,000,000 Da.

13. The method as claimed in any one of claims 1 to 12, wherein the method after the step of mixing and granulating the active ingredient, the lower alkyl alcohol solvent having the concentration not lower than 80% (w/v), and the cellulose derivate further comprises adding a solubilizer.

14. The method as claimed in claim 13, wherein the solubilizer comprises polyethylene glycol (PEG), docusate sodium, sodium lauryl sulfate, medium chain triglyceride, polyvinylpyrrolidone (PVP), xylitol, D-fructose, urea, acetylamine, polyoxyl 40 hydrogenated castor oil, polyoxyl 60 hydrogenated castor oil, poloxamer 407, benzoic acid, sodium benzoate, salicylic acid, sodium salicylate, or any combination thereof.

15. A sustained release pharmaceutical composition, **characterized in that** the sustained release pharmaceutical composition is obtained from the method for preparation according to any one of claims 1 to 14.

16. A sustained release pharmaceutical composition consisting of:

an active ingredient;
a cellulose derivate having viscosity from 50 mPa·s to 100,000 mPa·s and being hard to dissolve in a lower alkyl alcohol solvent having the concentration not lower than 80% (w/v);

the lower alkyl alcohol solvent having the concentration not lower than 80% (w/v) in an amount of less than 5% in weight (wt%) based on the total weight of the sustained release pharmaceutical composition;

a polyalkylene oxide having an average molecular weight above 500,000 Da; and

a lubricant.

17. The sustained release pharmaceutical composition as claimed in claim 16, wherein the polyalkylene oxide is un-granulated or un-sized.

18. The sustained release pharmaceutical composition as claimed in claim 16, wherein the ratio of the cellulose derivate and the polyalkylene oxide of the sustained release pharmaceutical composition is from 1:100 to 100:1.

19. The sustained release pharmaceutical composition as claimed in claim 16, based on the total weight percent (wt%) of the sustained release pharmaceutical composition, wherein:

the weight percent of the active ingredient is from 0.01 wt% to 25 wt%;

the weight percent of the cellulose derivate having the viscosity from 50 mPa·s to 100,000 mPa·s is from 1 wt% to 50 wt%;

the weight percent of the lower alkyl alcohol solvent having the concentration not lower than 80% (w/v) is from 0 wt% to 5 wt%;

the weight percent of the polyalkylene oxide having an average molecular weight above 500,000 Da is from 30 wt% to 95 wt%; and

the weight percent of the lubricant is from 0.1 wt% to 5 wt%.

20. The sustained release pharmaceutical composition as claimed in claim 19, wherein the sustained release pharmaceutical composition further comprises a solubilizer, which the weight percent is from 0 wt% to 10 wt%.

FIG. 1

FIG. 2

FIG. 3

FIG. 4

FIG. 5

Europäisches
Patentamt

European
Patent Office

Office européen
des brevets

# EUROPEAN SEARCH REPORT

**Application Number**

EP 16 17 1928

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/281906 A1 (KONDO HIROMU [JP] ET AL) 17 November 2011 (2011-11-17) * paragraphs [0126] - [0132] * * claims; examples * * paragraphs [0064] - [0072] * | 1-20 | INV. A61K9/16 A61K9/20 A61K31/00 |
| X | WO 2007/110878 A1 (PANACEA BIOTEC LTD [IN]; JAIN RAJESH [IN]; JINDAL KOUR CHAND [IN]; DEV) 4 October 2007 (2007-10-04) * page 9, line 4 - page 18, line 17 * * examples * * pages - * | 1-20 | |
| X | US 2011/008426 A1 (JAIN RAJESH [IN] ET AL) 13 January 2011 (2011-01-13) * the whole document * | 1-20 | |
| A | US 6 436 441 B1 (SAKO KAZUHIRO [JP] ET AL) 20 August 2002 (2002-08-20) * the whole document * | 1-20 | |
| A | EP 1 568 361 A2 (YAMANOUCHI PHARMA CO LTD [JP]) 31 August 2005 (2005-08-31) * the whole document * | 1-20 | TECHNICAL FIELDS SEARCHED (IPC) A61K |
| A | WO 2005/105036 A1 (NATCO PHARMA LTD [IN]; DURGA MAHESWARI PARVATANENI [IN]; APPALASWAMY N) 10 November 2005 (2005-11-10) * the whole document * | 1-20 | |

The present search report has been drawn up for all claims

| Place of search | Date of completion of the search | Examiner |
|---|---|---|
| The Hague | 18 October 2016 | Ceyte, Mathilde |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 17 1928

This annex lists the patent family members relating to the patent documents cited in the above-mentioned European search report.
The members are as contained in the European Patent Office EDP file on
The European Patent Office is in no way liable for these particulars which are merely given for the purpose of information.

18-10-2016

| Patent document cited in search report | | Publication date | Patent family member(s) | | Publication date |
|---|---|---|---|---|---|
| US 2011281906 | A1 | 17-11-2011 | CA | 2674039 A1 | 17-07-2008 |
| | | | EP | 2119442 A1 | 18-11-2009 |
| | | | US | 2009011018 A1 | 08-01-2009 |
| | | | US | 2011281906 A1 | 17-11-2011 |
| | | | WO | 2008084698 A1 | 17-07-2008 |
| WO 2007110878 | A1 | 04-10-2007 | AU | 2007230549 A1 | 04-10-2007 |
| | | | BR | PI0709222 A2 | 12-07-2011 |
| | | | CA | 2647421 A1 | 04-10-2007 |
| | | | CN | 101410096 A | 15-04-2009 |
| | | | CR | 10404 A | 08-12-2008 |
| | | | EA | 200870379 A1 | 28-04-2009 |
| | | | EP | 2004150 A1 | 24-12-2008 |
| | | | JP | 2009531420 A | 03-09-2009 |
| | | | MA | 30359 B1 | 01-04-2009 |
| | | | RS | 20080439 A | 06-05-2009 |
| | | | US | 2010234288 A1 | 16-09-2010 |
| | | | WO | 2007110878 A1 | 04-10-2007 |
| US 2011008426 | A1 | 13-01-2011 | AU | 2009220779 A1 | 11-09-2009 |
| | | | CA | 2717456 A1 | 11-09-2009 |
| | | | CN | 101969931 A | 09-02-2011 |
| | | | EA | 201071035 A1 | 29-04-2011 |
| | | | EP | 2262483 A2 | 22-12-2010 |
| | | | JP | 2011513391 A | 28-04-2011 |
| | | | KR | 20100126465 A | 01-12-2010 |
| | | | US | 2011008426 A1 | 13-01-2011 |
| | | | WO | 2009110005 A2 | 11-09-2009 |
| US 6436441 | B1 | 20-08-2002 | AT | 219933 T | 15-07-2002 |
| | | | AU | 682827 B2 | 23-10-1997 |
| | | | AU | 4983893 A | 12-04-1994 |
| | | | BG | 61861 B1 | 31-08-1998 |
| | | | BG | 99503 A | 31-01-1996 |
| | | | CA | 2144077 A1 | 31-03-1994 |
| | | | CN | 1091004 A | 24-08-1994 |
| | | | CZ | 9500679 A3 | 13-09-1995 |
| | | | DE | 69332081 D1 | 08-08-2002 |
| | | | DE | 69332081 T2 | 06-02-2003 |
| | | | DK | 0661045 T3 | 28-10-2002 |
| | | | EP | 0661045 A1 | 05-07-1995 |
| | | | ES | 2179829 T3 | 01-02-2003 |
| | | | FI | 951226 A | 16-03-1995 |
| | | | HU | 226456 B1 | 29-12-2008 |
| | | | JP | 3140465 B2 | 05-03-2001 |
| | | | KR | 100355130 B1 | 30-01-2003 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**

EP 16 17 1928

This annex lists the patent family members relating to the patent documents cited in the above-mentioned European search report.
The members are as contained in the European Patent Office EDP file on
The European Patent Office is in no way liable for these particulars which are merely given for the purpose of information.

18-10-2016

| Patent document cited in search report | | Publication date | Patent family member(s) | | | Publication date |
|---|---|---|---|---|---|---|
| | | | NO | 951036 | A | 17-03-1995 |
| | | | NZ | 255579 | A | 26-03-1996 |
| | | | PL | 308137 | A1 | 24-07-1995 |
| | | | PT | 661045 | E | 29-11-2002 |
| | | | RO | 112991 | B1 | 30-03-1998 |
| | | | RU | 2121830 | C1 | 20-11-1998 |
| | | | SK | 33895 | A3 | 08-11-1995 |
| | | | US | 6436441 | B1 | 20-08-2002 |
| | | | US | 6699503 | B1 | 02-03-2004 |
| | | | US | 2003203024 | A1 | 30-10-2003 |
| | | | WO | 9406414 | A1 | 31-03-1994 |
| EP 1568361 | A2 | 31-08-2005 | CZ | 15015 | U1 | 16-03-2005 |
| | | | EP | 1568361 | A2 | 31-08-2005 |
| | | | US | 2005100602 | A1 | 12-05-2005 |
| WO 2005105036 | A1 | 10-11-2005 | NONE | | | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- TW 104118277
- TW 393320
- TW 307632 I
- EP 1205190 A